# EUROPEAN PATENT APPLICATION

(11) **EP 3 831 328 A1**
(43) Date of publication of application: **09.06.2021**
(21) Application number: 20211879.0
(22) Date of filing: 04.12.2020
(51) Int. Cl.: A61B 34/10, A61B 34/20, A61B 1/00, A61B 1/267, A61B 90/00, G06T 7/00

(54) **METHOD FOR MAINTAINING LOCALIZATION OF DISTAL CATHETER TIP TO TARGET DURING VENTILATION AND/OR CARDIAC CYCLES**

(30) Priority: 04.12.2019 US 201962943696 P; 05.10.2020 US 202017062917
(71) Applicant: Covidien LP, Mansfield, MA 02048 (US)
(72) Inventor: GLEIMAN, Seth, Guilford, CT Connecticut 06437 (US)
(74) Representative: Maschio, Antonio

(57) **Abstract**

A method and system of maintaining a position of a catheter within a luminal network. The method including determining a position of a distal end of the catheter, determining a position of a target and calculating an offset between the distal end of the catheter and the target. The method further includes monitoring movement of the target and catheter, sending signals to one or more motors, the motors being in operable communication with and configured to adjust a distal portion of the catheter, and driving the one or more motors such that the offset between the distal end of the catheter and the target is substantially maintained.

## Description

### CROSS-REFERENCE TO RELATED APPLICATION

The present application claims the benefit of and priority to U.S. Provisional Patent Application Ser. No. 62/943,696, filed on December 4, 2019, the entire content of which is incorporated herein by reference.

### Technical Field

This disclosure relates to the field of navigation of and maintaining position of medical devices, such as biopsy or ablation tools, relative to targets.

### Description of Related Art

There are several commonly applied medical methods, such as endoscopic procedures or minimally invasive procedures, for treating various maladies affecting organs including the liver, brain, heart, lungs, gall bladder, kidneys, and bones. Often, one or more imaging modalities, such as magnetic resonance imaging (MRI), ultrasound imaging, computed tomography (CT), or fluoroscopy are employed by clinicians to identify and navigate to areas of interest within a patient and ultimately a target for biopsy or treatment. In some procedures, pre-operative scans may be utilized for target identification and intraoperative guidance. However, real-time imaging may be required to obtain a more accurate and current image of the target area. Furthermore, real-time image data displaying the current location of a medical device with respect to the target and its surroundings may be needed to navigate the medical device to the target in a safe and accurate manner (e.g., without causing damage to other organs or tissue).

For example, an endoscopic approach has proven useful in navigating to areas of interest within a patient, and particularly so for areas within luminal networks of the body such as the lungs. To enable the endoscopic approach, and more particularly the bronchoscopic approach in the lungs, endobronchial navigation systems have been developed that use previously acquired MRI data or CT image data to generate a three-dimensional (3D) rendering, model, or volume of the particular body part such as the lungs.

The resulting volume generated from the MRI scan or CT scan may be utilized to create a navigation plan to facilitate the advancement of a navigation catheter (or other suitable medical device) through a bronchoscope and a branch of the bronchus of a patient to an area of interest. A locating or tracking system, such as an electromagnetic (EM) tracking system, may be utilized in conjunction with, for example, CT data, to facilitate guidance of the navigation catheter through the branch of the bronchus to the area of interest. In certain instances, the navigation catheter may be positioned within one of the airways of the branched luminal networks adjacent to, or within, the area of interest to provide access for one or more medical instruments.

However, once a catheter is navigated to a desired location the position of the catheter within the patient is constantly in flux. Change in position of the catheter may be caused by the movement of tools through the catheter, movement of the lungs themselves during respiration, and movement caused by the proximity of the lungs to the heart which is in constant motion as part of the cardiac process. Accordingly, improvements to current systems are desired.

### SUMMARY

One aspect of the disclosure is directed to a method of maintaining a position of a catheter including: determining a position of a distal end of the catheter, determining a position of a target, calculating an offset between the distal end of the catheter and the target, monitoring movement of the target and catheter. The method also includes sending signals to one or more motors, the motors being in operable communication with and configured to adjust a distal portion of the catheter. The method also includes driving the one or more motors such that the offset between the distal end of the catheter and the target is substantially maintained. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The method further including conducting a local registration of the catheter and the target. The method further including capturing a fluoroscopic image of the target and the catheter. The method further including comparing the fluoroscopic image to slices of a fluoroscopic 3D reconstruction captured during the local registration. The method including detecting the position of the target using ultrasonic visualization. The method further including detecting a position of a distal end of the catheter with a sensor. The method where the sensor is an electromagnetic sensor. The method where the position of the target is calculated based on the calculated offset and the detected position of the distal end of the catheter. Implementations of the described techniques may include hardware, a method or process, or computer software on a computer-accessible medium, including software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

A further aspect of the disclosure is directed to a method of navigating a catheter to a desired location within a luminal network including: receiving a CT image data set, identifying one or more targets in the CT image data set, generating a three-dimensional (3D) model of the luminal network and pathway to the one or more targets, registering the 3D model and pathway to a luminal network, updating the position of the catheter in the 3D model as the along the pathway proximate one of the targets, performing a local registration to determine a relative position of the catheter and one of the targets, acquiring a fluoroscopic image of the luminal network, determining a position of the target and the catheter in the fluoroscopic image, detecting a position of a sensor on a distal portion of the catheter, calculating a position of the target in a coordinate system of the sensor, and receiving signals from a computing device to drive one or more motors operably associated with the catheter to maintain the catheter position relative to the target position constant. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The method further including comparing the fluoroscopic image to slices of a fluoroscopic 3D reconstruction captured during the local registration. The method including detecting the position of the target using ultrasonic visualization. The method where the sensor is operably connected to a distal portion of a catheter. The method where the sensor is an electromagnetic sensor. The method further including detecting a position of one or more reference sensors. The method where the fluoroscopic image is a fluoroscopic video, and the calculating a position of the target in a coordinate system of the sensor in undertaken multiple times through the fluoroscopic video. The method further including matching movement of the target determined by repeatedly determining of the target's position with the movement of the one or more reference sensor and the sensor on the distal portion of the catheter. The method further including driving the one or more motors based on the received driving signals to maintain the catheter position relative to the target position constant when the movement of the one or more reference sensor or the sensor on the distal portion of the catheter is within a tolerance limit. The method including detecting that the position of the reference sensor or sensor on the distal portion of the catheter is outside of a tolerance limit and performing a second local registration to determine a new relative position of the catheter and one of the targets, acquiring a further fluoroscopic image of the luminal network, determining a new position of the target and the catheter in the fluoroscopic image, detecting a new position of a sensor on a distal portion of the catheter, calculating a new position of the target in a coordinate system of the sensor, and receiving signals from a computing device to drive one or more motors operably associated with the catheter to maintain the new catheter position relative to the new target position constant. The method where the position of the target is calculated based on the calculated offset and the detected position of the distal end of the catheter. Implementations of the described techniques may include hardware, a method or process, or computer software on a computer-accessible medium, including software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

Still a further aspect of the disclosure is directed to a system including: a catheter including a drive mechanism to manipulate a distal portion of the catheter, the catheter including a sensor on a distal portion thereof; a computing device including a processor and a computer readable recording medium, the computing device configured to receive signals from the sensor to determine a position of the distal portion of the catheter. The system also includes receive signals from one or more reference sensors. The system also includes receive a fluoroscopic video of the catheter proximate a target. The system also includes determine a relative position of the catheter and the target at multiple instances in the fluoroscopic video. The system also includes calculate a position of the target in a coordinate system of the sensor. The system also includes generate signals to drive the distal portion of the catheter to maintain the catheter position relative to the target position constant. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The system where the drive mechanism includes one or more motors operably connected to one or more pull wires and configured to receive the signals generated by the computing device. The system where in the sensor on the distal portion of the catheter is an electromagnetic sensor. The system where the drive mechanism is part of a robotic catheter drive system. Implementations of the described techniques may include hardware, a method or process, or computer software on a computer-accessible medium, including software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

### BRIEF DESCRIPTION OF THE DRAWINGS

Various aspects and embodiments of the disclosure are described hereinbelow with references to the drawings, wherein:
FIG. 1 is a schematic diagram of a system for navigating to soft-tissue targets via luminal networks in accordance with the disclosure;
FIG. 2 is a user interface of a navigation program in accordance with aspects of the disclosure;
FIG. 3 is a user interface of a navigation program in accordance with aspects of the disclosure;
FIG. 4 is a user interface of a navigation program in accordance with aspects of the disclosure;
FIG. 5 is a user interface of a navigation program in accordance with aspects of the disclosure;
FIG. 6 is a user interface of a navigation program in accordance with aspects of the disclosure;
FIG. 7 is a user interface of a navigation program in accordance with aspects of the disclosure;
FIG. 8 is a user interface of a navigation program in accordance with aspects of the disclosure;
FIG. 9 is a user interface of a navigation program in accordance with aspects of the disclosure;
FIG. 10 is a user interface of a navigation program in accordance with aspects of the disclosure;
FIG. 11 is a flow chart detailing a method in accordance with the disclosure;
FIG. 12A is a perspective view of a motorized catheter in accordance with the disclosure;
FIG. 12B is a detailed magnified view of a portion of the drive mechanism of the motorized catheter of FIG. 12A;
FIG. 13 is a user interface of an ultrasound imaging application; and

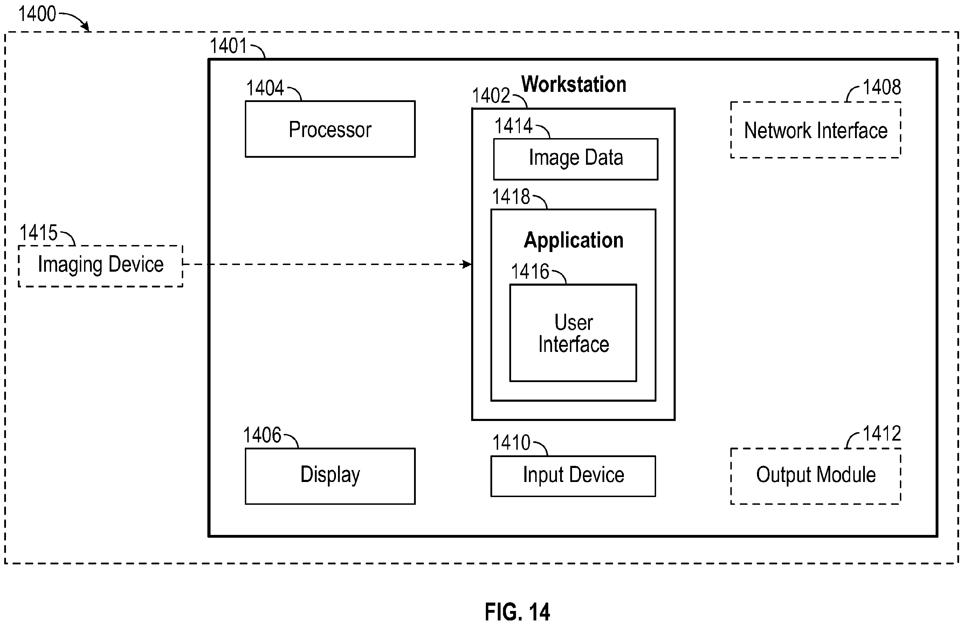

FIG. 14 is a schematic view of a computing device in accordance with aspects of the disclosure.

### DETAILED DESCRIPTION

In accordance with the disclosure, a 3D volume of a patient's lungs or another suitable portion of the anatomy, may be generated from previously acquired scans, such as CT scans. These scans may be used to generate a 3D model of the anatomy. The 3D model and related scan data are used to identify targets, e.g., potential lesions for biopsy or treatment, and to generate a pathway plan through the anatomy to reach the targets.

Once the pathway plan is generated and accepted by a clinician, that pathway plan may be utilized by a navigation system to drive a catheter along the pathway plan through the anatomy to reach the desired target. The driving of the catheter along the pathway plan may be manual or it may be robotic, or a combination of both. Manual systems include the ILLUMISITE navigation system sold by Medtronic PLC, robotic systems include the ION system sold by Intuitive Surgical Inc. and the MONARCH system sold by Auris Health, Inc. In a single procedure planning, registration of the pathway plan to the patient, and navigation are performed to enable a medical device, e.g., a catheter to be navigated along the planned path to reach a target, e.g., a lesion, so that a biopsy or treatment of the target can be completed.

As noted above, whether manual or robotic, the pathway plan and 3D model developed from the pre-procedure scan data must be registered to the patient before navigation of the catheter to a target within the anatomy can begin. Once registered, a catheter or other tool may be navigated following the pathway plan to a desired location. While this registration is generally more that suitable for general navigation of the pathway, regardless of the registration method employed, and there are numerous registration methods, the 3D model and pathway plan may still not provide sufficient accuracy for the "last mile" of navigation allowing for the guidance of medical devices or instruments into the target for biopsy and treatment.

In some cases, the inaccuracy is caused by deformation of the patient's lungs during the procedure relative to the lungs at the time of the acquisition of the previously acquired CT data. This deformation (CT-to-Body divergence) may be caused by many different factors including, for example, changes in the body when transitioning from between a sedated state and a non-sedated state, the bronchoscope changing the patient's pose, the bronchoscope and catheter pushing the tissue, different lung volumes (e.g., the CT scans are acquired during full breath hold following inhale while navigation is typically performed while the patient is breathing), different beds, different days, etc. Thus, another imaging modality may be employed to assist in visualizing medical devices and targets in real-time and enhance the in-vivo navigation procedure.

In navigating the medical device to the target, clinicians may use a fluoroscopic imaging to visualize the position of the medical device relative to the target. While fluoroscopic images show highly dense objects, such as metal tools, bones, and large soft-tissue objects, e.g., the heart, the fluoroscopic images may not clearly show small soft-tissue objects of interest, such as lesions. Furthermore, the fluoroscopic images are two-dimensional (2D) projections which makes determining depths in the view difficult.

X-ray volumetric reconstruction has been developed to enable identification of soft tissue objects and to update the relative position of the target and the catheter in the pathway plan and 3D model. The volumetric reconstruction is made from a series of 2D fluoroscopic images taken at different angles to the tissue in question. In one method described in greater detail below, updating of the pathway plan and relative positions of the catheter and target can be achieved with a local registration process. This local registration process reduces CT-to-body divergence. After the local registration process, in one embodiment a locatable guide (i.e., a catheter with multiple sensors) may be removed from the catheter and a medical device, e.g., a biopsy tool, is introduced into the catheter for navigation to the target to perform the biopsy or treatment of the target, e.g., the lesion.

However, even with local registration where the relative position of the catheter and the target is updated in the 3D model and the pathway plan, maintaining the alignment of the catheter and the target as confirmed in the local registration can be challenging. The source of this challenge is related to two primary functions of the body, namely respiration and cardiac functions (i.e., heartbeat).

Another source of errors is the passage of tools through the catheter after local registration can cause tip deflection. When the catheter includes a sensor, e.g., an electromagnetic sensor or a flexible sensor (sensing shape and orientation of a portion of the catheter) these types of movements can be reported to the clinician via a graphic user interface (GUI) on which navigation software is displayed and allows for following the pathway plan to the target. Movements caused by the passage of tools through the catheter appear as movement of the target relative the position of the catheter on the GUI.

One embodiment of the disclosure is directed to a catheter including one or more pull wires. The movement of the sensor is sensed by the navigation system and micro-adjustments can be made to the location of the catheter by manipulating the pull wires. These same pull wires can be employed to constantly adjust the position of the catheter, and specifically the distal portion of the catheter as it moves as a result of lung motion caused by the cardiac cycle or ventilation cycle. This adjustment can eliminate the need for the clinician to have the patient, undertake a "breath hold" during the procedure to at least minimize the movement of the lungs during portions of the procedure. As is known breath holds take time and can place stress on the patient if the breath hold is held for too long.

The pull wires cause the catheter to change shape and curvature at the distal portion and may be manipulated either manually by a clinician or automatically via a computer-controlled system (e.g., a robot). Where computer controlled, the sensed position of the catheter is used as the input to the robot can create a "phase lock" where the relative position of the catheter and the target are kept constant through constant manipulation of the pull wires.

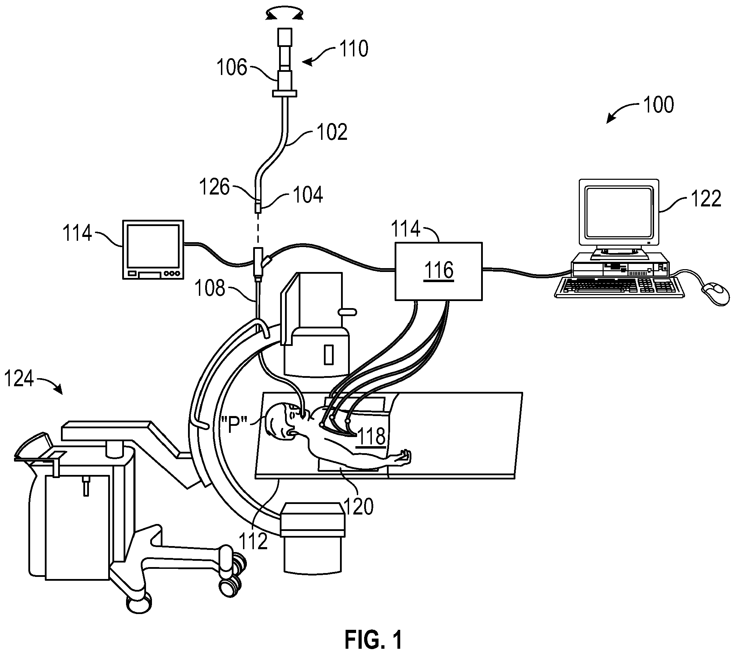

In accordance with aspects of the disclosure, and as noted above, the visualization of intra-body navigation of a medical device, e.g., a biopsy tool, towards a target, e.g., a lesion, may be a portion of a larger workflow of a navigation system, such as an electromagnetic navigation system. FIG. 1 is a perspective view of an exemplary system for facilitating navigation of a medical device, e.g., a catheter to a soft-tissue target via airways of the lungs. System 100 may be further configured to construct fluoroscopic based three-dimensional volumetric data of the target area from 2D fluoroscopic images to confirm navigation to a desired location. System 100 may be further configured to facilitate approach of a medical device to the target area by using Electromagnetic Navigation (EMN) and for determining the location of a medical device with respect to the target. One such EMN system is the ILLUMISITE system currently sold by Medtronic PLC, though other systems for intraluminal navigation are considered within the scope of the disclosure, as noted above.

One aspect of the system 100 is a software component for reviewing of computed tomography (CT) image scan data that has been acquired separately from system 100. The review of the CT image data allows a user to identify one or more targets, plan a pathway to an identified target (planning phase), navigate a catheter 102 to the target (navigation phase) using a user interface on computing device 122, and confirming placement of a sensor 104 relative to the target. The target may be tissue of interest identified by review of the CT image data during the planning phase. Following navigation, a medical device, such as a biopsy tool or other tool, may be inserted into catheter 102 to obtain a tissue sample from the tissue located at, or proximate to, the target.

As shown in FIG. 1, catheter 102 is part of a catheter guide assembly 106. In practice, catheter 102 is inserted into a bronchoscope 108 for access to a luminal network of the patient P. Specifically, catheter 102 of catheter guide assembly 106 may be inserted into a working channel of bronchoscope 108 for navigation through a patient's luminal network. A locatable guide (LG) 110 (a second catheter), including a sensor 104 is inserted into catheter 102 and locked into position such that sensor 104 extends a desired distance beyond the distal tip of catheter 102. The position and orientation of sensor 104 relative to a reference coordinate system, and thus the distal portion of catheter 102, within an electromagnetic field can be derived. Catheter guide assemblies 106 are currently marketed and sold by Medtronic PLC under the brand names SUPERDIMENSION® Procedure Kits, or EDGE™ Procedure Kits, and are contemplated as useable with the disclosure.

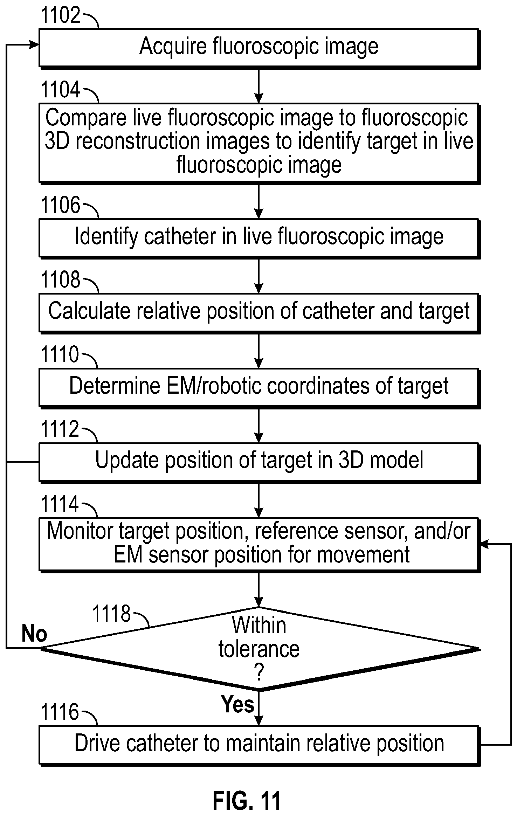

System 100 generally includes an operating table 112 configured to support a patient P, a bronchoscope 108 configured for insertion through patient P's mouth into patient P's airways; monitoring equipment 114 coupled to bronchoscope 108 (e.g., a video display, for displaying the video images received from the video imaging system of bronchoscope 108); a locating or tracking system 114 including a locating module 116, a plurality of reference sensors 18 and a transmitter mat 120 including a plurality of incorporated markers; and a computing device 122 including software and/or hardware used to facilitate identification of a target, pathway planning to the target, navigation of a medical device to the target, and/or confirmation and/or determination of placement of catheter 102, or a suitable device therethrough, relative to the target. Computing device 122 may be similar to workstation 1401 of FIG. 140 and may be configured to execute the methods of the disclosure including the method of FIG. 11.

A fluoroscopic imaging device 124 capable of acquiring fluoroscopic or x-ray images or video of the patient P is also included in this particular aspect of system 100. The images, sequence of images, or video captured by fluoroscopic imaging device 124 may be stored within fluoroscopic imaging device 124 or transmitted to computing device 122 for storage, processing, and display. Additionally, fluoroscopic imaging device 124 may move relative to the patient P so that images may be acquired from different angles or perspectives relative to patient P to create a sequence of fluoroscopic images, such as a fluoroscopic video. The pose of fluoroscopic imaging device 124 relative to patient P and while capturing the images may be estimated via markers incorporated with the transmitter mat 120. The markers are positioned under patient P, between patient P and operating table 112 and between patient P and a radiation source or a sensing unit of fluoroscopic imaging device 124. The markers incorporated with the transmitter mat 120 may be two separate elements which may be coupled in a fixed manner or alternatively may be manufactured as a single unit. Fluoroscopic imaging device 124 may include a single imaging device or more than one imaging device.

Computing device 122 may be any suitable computing device including a processor and storage medium, wherein the processor is capable of executing instructions stored on the storage medium. Computing device 122 may further include a database configured to store patient data, CT data sets including CT images, fluoroscopic data sets including fluoroscopic images and video, fluoroscopic 3D reconstruction, navigation plans, and any other such data. Although not explicitly illustrated, computing device 122 may include inputs, or may otherwise be configured to receive, CT data sets, fluoroscopic images/video and other data described herein. Additionally, computing device 122 includes a display configured to display graphical user interfaces. Computing device 122 may be connected to one or more networks through which one or more databases may be accessed.

With respect to the planning phase, computing device 122 utilizes previously acquired CT image data for generating and viewing a three-dimensional model or rendering of patient P's airways, enables the identification of a target on the three-dimensional model (automatically, semiautomatically, or manually), and allows for determining a pathway through patient P's airways to tissue located at and around the target. More specifically, CT images acquired from previous CT scans are processed and assembled into a three-dimensional CT volume, which is then utilized to generate a three-dimensional model of patient P's airways. The three-dimensional model may be displayed on a display associated with computing device 122, or in any other suitable fashion. Using computing device 122, various views of the three-dimensional model or enhanced two-dimensional images generated from the three-dimensional model are presented. The enhanced two-dimensional images may possess some three-dimensional capabilities because they are generated from three-dimensional data. The three-dimensional model may be manipulated to facilitate identification of target on the three-dimensional model or two-dimensional images, and selection of a suitable pathway through patient P's airways to access tissue located at the target can be made. Once selected, the pathway plan, three-dimensional model, and images derived therefrom, can be saved and exported to a navigation system for use during the navigation phase(s). The ILLUMISITE software suite currently sold by Medtronic PLC includes one such planning software.

With respect to the navigation phase, a six degrees-of-freedom electromagnetic locating or tracking system 114, or other suitable system for determining position and orientation of a distal portion of the catheter 102, is utilized for performing registration of the images and the pathway for navigation. Tracking system 114 includes the tracking module 116, a plurality of reference sensors 118, and the transmitter mat 120 (including the markers). Tracking system 114 is configured for use with a locatable guide 110 and particularly sensor 104. As described above, locatable guide 110 and sensor 104 are configured for insertion through catheter 102 into patient P's airways (either with or without bronchoscope 108) and are selectively lockable relative to one another via a locking mechanism.

Transmitter mat 120 is positioned beneath patient P. Transmitter mat 120 generates an electromagnetic field around at least a portion of the patient P within which the position of a plurality of reference sensors 118 and the sensor 104 can be determined with use of a tracking module 116. A second electromagnetic sensor 126 may also be incorporated into the end of the catheter 102. The second electromagnetic sensor 126 may be a five degree-of-freedom sensor or a six degree-of-freedom sensor. One or more of reference sensors 118 are attached to the chest of the patient P. Registration is generally performed to coordinate locations of the three-dimensional model and two-dimensional images from the planning phase, with the patient P's airways as observed through the bronchoscope 108, and allow for the navigation phase to be undertaken with knowledge of the location of the sensor 104.

Registration of the patient P's location on the transmitter mat 120 may be performed by moving sensor 104 through the airways of the patient P. More specifically, data pertaining to locations of sensor 104, while locatable guide 110 is moving through the airways, is recorded using transmitter mat 120, reference sensors 118, and tracking system 114. A shape resulting from this location data is compared to an interior geometry of passages of the three-dimensional model generated in the planning phase, and a location correlation between the shape and the three-dimensional model based on the comparison is determined, e.g., utilizing the software on computing device 122. In addition, the software identifies non-tissue space (e.g., air filled cavities) in the three-dimensional model. The software aligns, or registers, an image representing a location of sensor 104 with the three-dimensional model and/or two-dimensional images generated from the three-dimension model, which are based on the recorded location data and an assumption that locatable guide 110 remains located in non-tissue space in patient P's airways. Alternatively, a manual registration technique may be employed by navigating the bronchoscope 108 with the sensor 104 to pre-specified locations in the lungs of the patient P, and manually correlating the images from the bronchoscope to the model data of the three-dimensional model.

Though described herein with respect to EMN systems using EM sensors, the instant disclosure is not so limited and may be used in conjunction with flexible sensor, ultrasonic sensors, or without sensors. Additionally, the methods described herein may be used in conjunction with robotic systems such that robotic actuators drive the catheter 102 or bronchoscope 108 proximate the target.

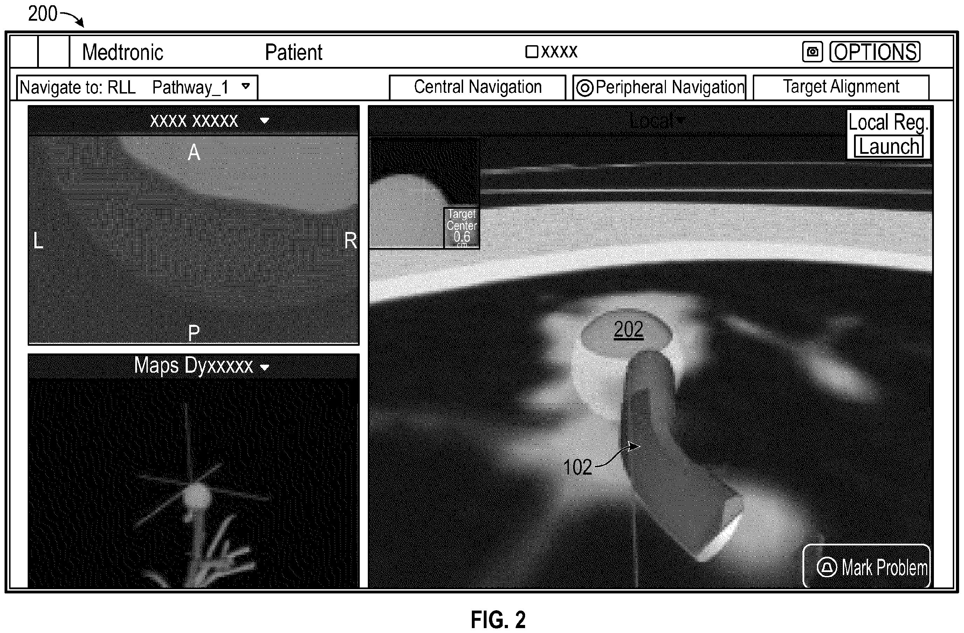

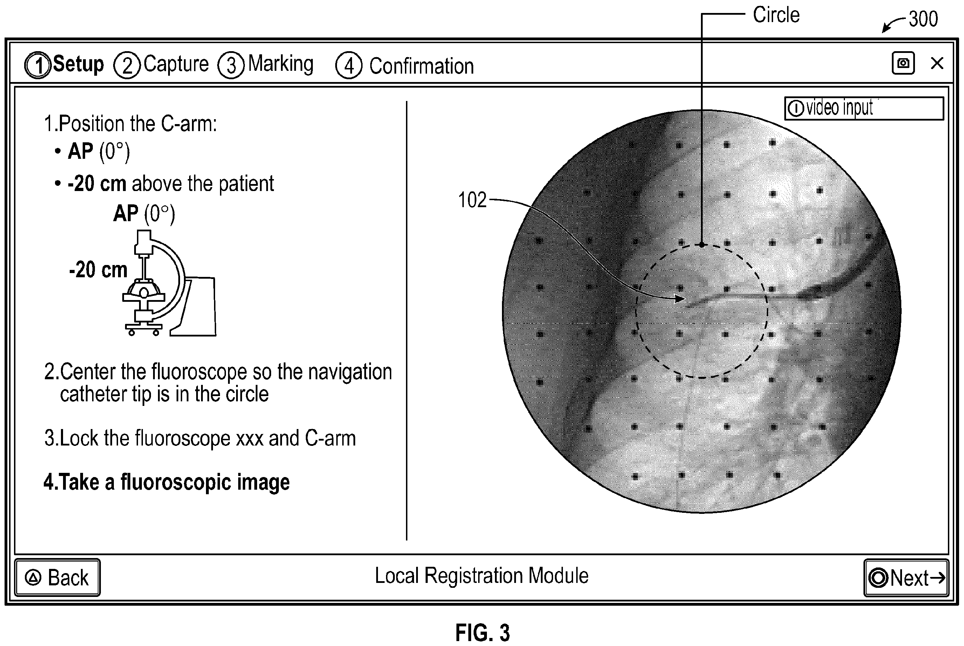

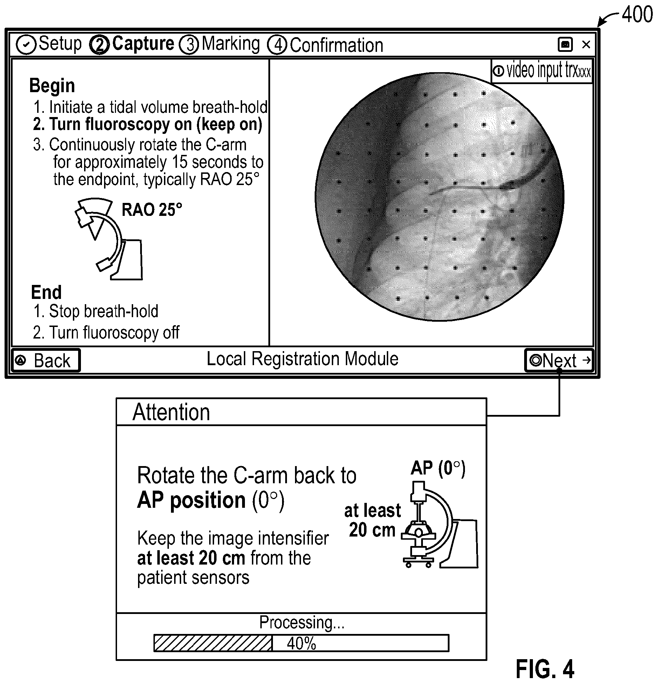

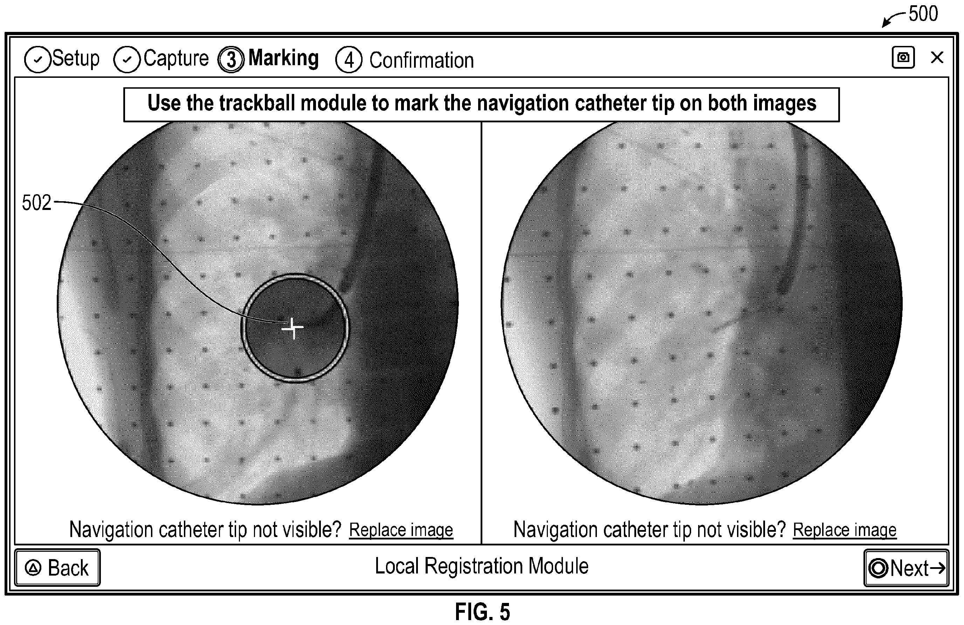

Following registration of the patient P to the image data and pathway plan, a user interface 200 as shown in Fig. 2 is displayed on the computing device 122 using the navigation software which sets forth the pathway that the clinician is to follow to reach the target. Once catheter 102 has been successfully navigated proximate, as shown in Fig. 2, the target 202 a local registration process may be performed for each target to reduce the CT-to-body divergence. An initial step is to acquire a 2D fluoroscopic image of the catheter 102 and mark the area of the catheter 102 as shown in Fig. 3, for example by arranging a circle such that the end of the catheter 102 is proximate the center of the circle. Next, as depicted in Fig. 4, a sequence of fluoroscopic images captured via fluoroscopic imaging device 124 for example from about 25 degrees on one side of the AP position to about 25 degrees on the other side of the AP position. A fluoroscopic 3D reconstruction may be then generated by the computing device 122. The generation of the fluoroscopic 3D reconstruction is based on the sequence of fluoroscopic images and the projections of structure of markers incorporated with transmitter mat 120 on the sequence of images. Following generation of the fluoroscopic 3D reconstruction, two fluoroscopic images are displayed in the GUI on computing device 122, as shown in Fig. 5. As depicted the GUI 500 in Fig. 5, the end of the catheter 102 needs to be marked using a marker 502 in each of these images. The two images are taken from different portions of the fluoroscopic 3D reconstruction. The fluoroscopic images of the 3D reconstruction may be presented on the user interface in a scrollable format where the user is able to scroll through the slices in series if desired.

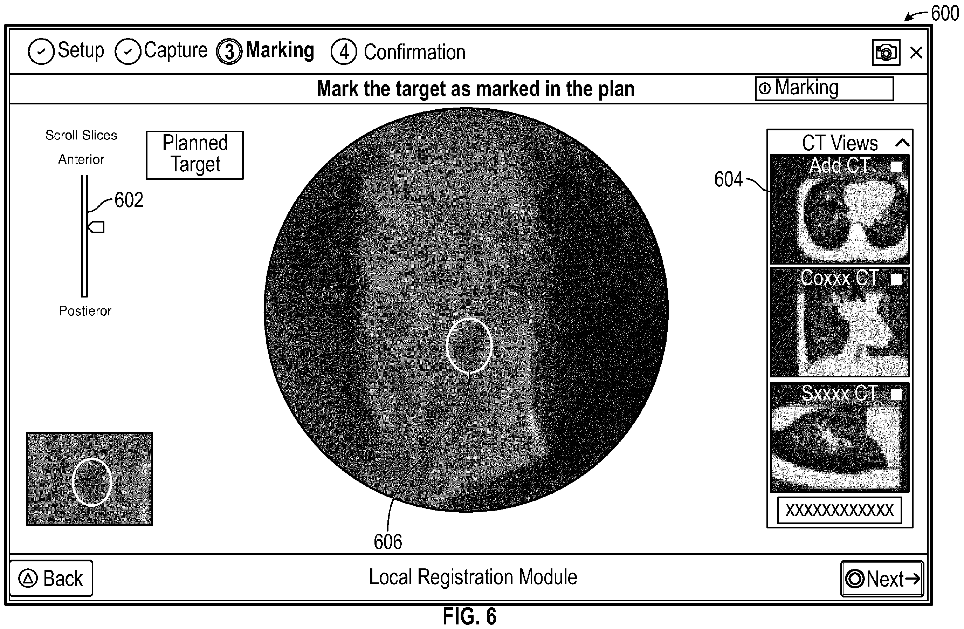

Next as depicted in Fig. 6, the clinician is directed to identify and mark the target in the fluoroscopic 3D reconstruction in GUI 600. A scroll bar 602 allows the clinician to scroll through the fluoroscopic 3D reconstruction until the target is identified. CT views 604 may also be displayed to assist in identifying the position of the target. Once identified, a marker 606 is placed on the target.

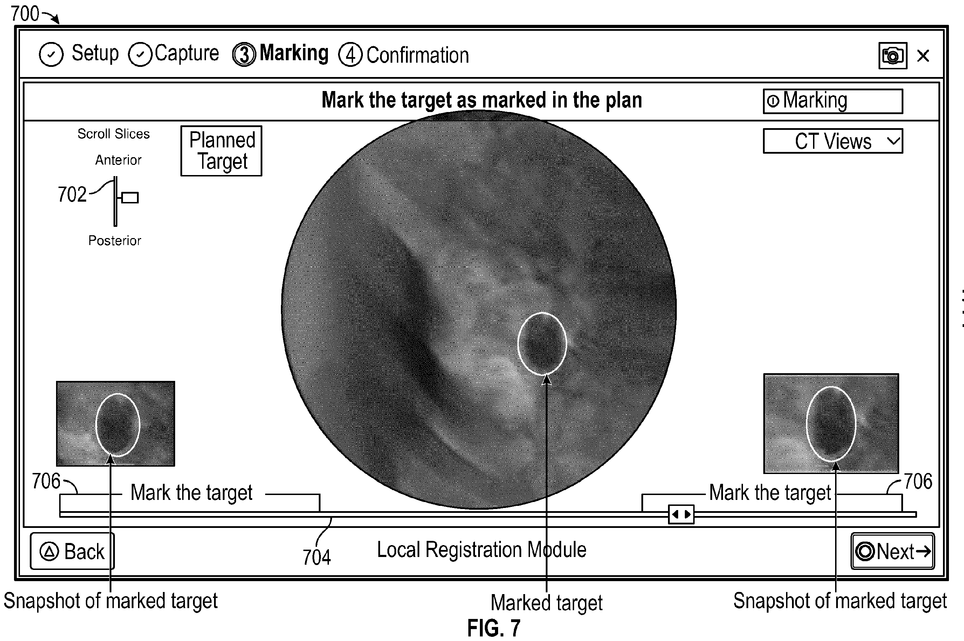

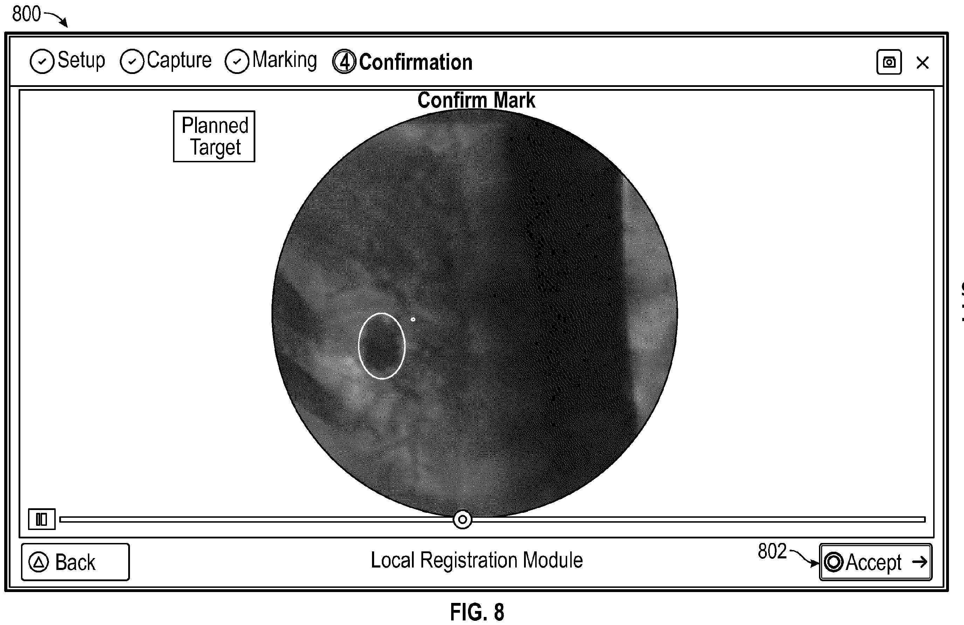

Following the marking of the target in GUI 600, the clinician will be asked to mark the target in two different perspectives in GUI 700 of Fig. 7. Again, a scroll bar 702 allows for changes of the depth of the fluoroscopic image being displayed so the target can be found in the fluoroscopic 3D reconstruction. A second scrollbar 704 allows for translation or rotation though the fluoroscopic 3D reconstruction to reach the two perspectives in which the target is to be marked. The portion of the fluoroscopic 3D reconstruction in which the target is to be marked is identified by zone markers 706. Once the target is marked in each perspective, a thumbnail image of that perspective with the target marked is displayed proximate the zone markers 706. Once complete, the clinician is presented with a GUI 800 in FIG. 8 where the entire fluoroscopic 3D reconstruction may be viewed to ensure that the target or lesion remains within the marker 802 through the fluoroscopic 3D reconstruction.

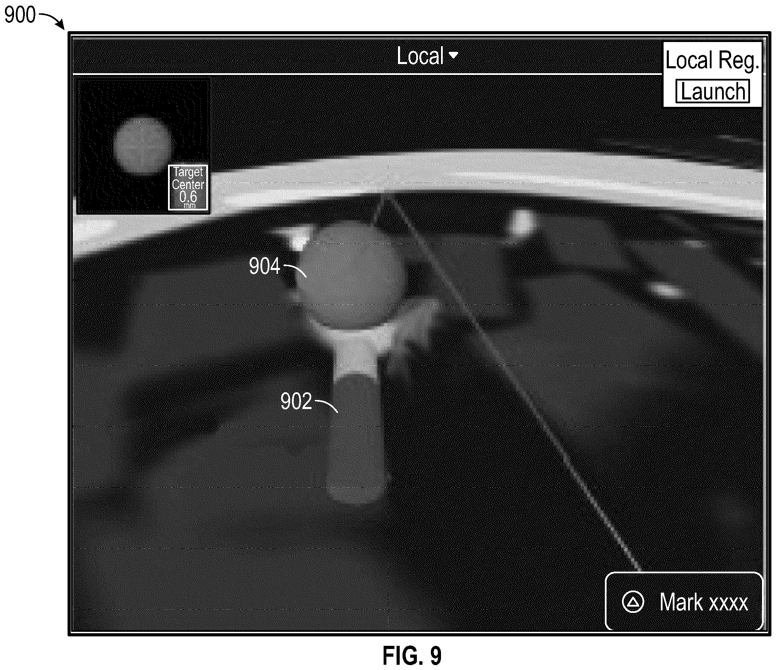

After confirming that there are marks on the target throughout the fluoroscopic 3D reconstruction, the clinician may select the "Accept" button 802, at which point the local registration process ends and the relative position of the catheter 102 in the 3D model and the pathway plan is updated to display the actual current relative position of the end of the catheter 102 and the target. By the local registration process the offset between the location of the target and the tip of the catheter 102 is determined as they are observed in the fluoroscopic 3D reconstruction. The offset is utilized, via computing device 122, to correct any errors in the original registration process and minimize any CT-to-body divergence. As a result, the location and/or orientation of the navigation catheter on the GUI with respect to the target is updated. This update is seamless to the clinician, and a GUI 900 is presented in computer device 122 as depicted in Fig. 9 with the updated relative positions of the location of the catheter 102 represented by a virtual catheter 902 and the target 904. At this point the clinician has a high degree of confidence that the catheter 102 is at the location relative to the target as displayed in the GUI 900.

By the process described above the relative positions of the catheter 102 and the target are marked in the 3D fluoroscopic reconstruction and the offset determined. In addition, the position of the catheter 102 is always being sensed either in the EM field to provide EM coordinates of its position, or in robotic coordinates if a robot is employed. As a result of the combination of the offset with the detection of position of the catheter 102, EM field coordinates or robotic coordinates of the target can be defined.

The relative position of the catheter 102 and target as shown in Fig. 9 is typically determined while the patient is at a breath hold condition to minimize the movement of the catheter 102 in the fluoroscopic imaging. Accordingly, the local registration does not account for movement of the catheter 102 or the target caused by respiration or heartbeat. Further, after the local registration process, the clinician or robot will typically remove the LG 110 with sensor 104 from the catheter 102 and insert a medical device in the catheter 102 and advance the medical device towards the target. As will be appreciated, in addition to the effects of respiration and heartbeat the relative positions of the catheter 102 and the target can be affected by the removal of the LG and the insertion of other tools. Thus, while the local registration is an improvement and overcomes the CT to body divergence, it is necessarily a static update to the relative positions of the catheter 102 and the target.

A target tracking mechanism employing live fluoroscopy in accordance with the disclosure can be used to determine the relative position of the catheter 102 and the target in real time. Further, this real time determination of the catheter 102 and the target can be employed to generate signals to adjust the position of the catheter 102 to maintain a relative position between the catheter 102 and target where the catheter includes a pull-wire positioning mechanism, described further below.

The target tracking mechanism utilizes the identification of the target in the local registration process, as described above with respect to Fig. 7 as a starting point. Once the target has been identified in the fluoroscopic 3D reconstruction the general contours and shape of the target can be analyzed by an image processing application running on computing device 122.

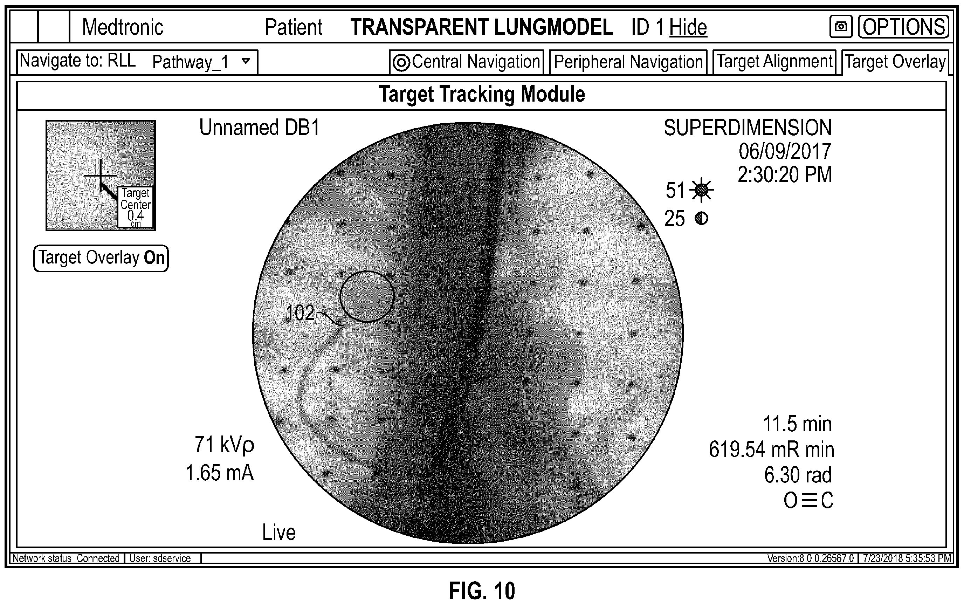

At any point following the local registration, which eliminates the CT-to-body divergence, a target tracking module may be engaged as described with reference to Fig. 11. As one example the target tracking module may be engaged following removal of the LG and insertion of a biopsy tool. The target tracking module starts at step 1102 by acquiring a live fluoroscopic image as depicted in Fig. 10. Next at step 1104 an image processing application running on the computing device 122 can compare the live fluoroscopic image to the images acquired in generating the fluoroscopic 3D reconstruction and using a pixel comparison identify the target 1002 in the live fluoroscopic image, as shown in Fig. 10. Next, the image processing application can identify the distal portion of the catheter 102 in the live fluoroscopic image in Fig. 10 at step 1106. At step 1108 the relative position of the catheter 102 and the target in the fluoroscopic image can be calculated. The position of the catheter 102 in EM coordinates is constantly obtained from the EMN system. With the relative position of the catheter 102 and target determined by the image processing application and the position of the catheter in EM coordinates identified by the EMN system, a position of the target in EM coordinates can be determined at step 1110. Where the live fluoroscopic image is a fluoroscopic video, this analysis and determination of the relative position of the catheter 102 and the target can be undertaken at a high frequency during the acquisition of the fluoroscopic video. Once the real-time position of the target is determined in EM coordinates is determined this position can be used to update the position at which the target is displayed in the 3D model (e.g. Fig. 10) relative to the detected position of the catheter at 1112.

In another aspect of the disclosure, because the contours of the target were identified in three dimensions during the local registration, the identification of the target in any single 2D fluoroscopic image can be matched to a slice of the three-dimensional target. By matching the target in the 2D fluoroscopic image to a slice of the 3D target from the local registration a position of the entirety of the target in EM coordinates (i.e., in three-dimensions) can be determined, and that position used to update the position of the target in the 3D model relative to the catheter 102. As noted above, this procedure can be undertaken at any point in the procedure and may be performed multiple times. Further, as long as live fluoroscopic images are being acquired, the position of the target can be constantly tracked in the images as it moves as a result of the respiration and the EM coordinates of the target constantly monitored.

In a further aspect of the disclosure, the movement in EM coordinates of the target can be monitored of a specified time period (e.g., 30 seconds) at step 1114. During the specified time period a number of respiration cycles and a number of cardiac cycles are captured, and the movement of the target as a result of these bodily functions be observed in the fluoroscopic images and the changes in EM coordinates determined as outlined above. This monitoring will require the acquisition of live fluoroscopic images (e.g., a video) for the specified time period. The detected movement of the target during this sampling can serve as a baseline expected movement of the target during normal respiration and cardiac signals. During this same time period the position of the reference sensors 118 can be monitored. The observed movement of the target in the fluoroscopic images as a result of respiration and cardiac cycles can be matched to the movement of the reference sensors 118. In one aspect of the disclosure, following the matching, if there are no out-of-tolerance movements of the reference sensors 118 detected, then the position of the target during respiration and cardiac signals can be assumed to correspond to the position it was at when the position of the target was matched to the position of the reference sensor 118 based on the detected position of the reference sensors 118. This determined position can be displayed on the UI 900. Further, as described below, the movements of the catheter 102 and the target during the time period can be used to drive the catheter 102 in such a way that the offset between them is maintained at all times in the cardiac and respiratory cycle at step 1116. If, however, the patient coughs or there is some other event that causes movement of the reference sensors 118 to move outside of a tolerance or because of the exchange of tools in the catheter 102 it position of the catheter 102 is outside of a tolerance as detected at step 1118, the computing system 122 can alert the clinician that the baseline movement is no longer valid and a further fluoroscopic video of the target may be required to accurately determine the position of the target in EM coordinates.

Still a further aspect of the disclosure relates to the catheter 102. The catheter 102 may include one or more pull-wires which can be used to manipulate the distal portion of the catheter. Pull-wire systems are known and used in a variety of settings including robotically assisted surgeries. In most catheter-based pull-wire systems at least one but up to six and even ten pull wires are incorporated into the catheter 102 and extend from proximate the distal end to a drive mechanism located at a proximal end. By tensioning and relaxing the pull-wires the shape of the distal portion of the catheter can be manipulated. For example, in a simple two pull-wire system by relaxing one pull-wire and retracting an opposing pull-wire the catheter may be deflected in the direction of the retracting pull-wire. Though pull-wire systems are described here in detail, the disclosure is not so limited, and the manipulation of the catheter 102 may be achieved by a variety of means including concentric tube systems and others that enable movement of the distal end of the catheter 102.

In procedures such as lung biopsy and treatment it is useful to ensure that the distal end of the catheter 102 is pointed directly at the target. In accordance with one aspect of the disclosure, the position of the target is detected and monitored as described above. Further, the position of the catheter 102 may be adjusted by a drive mechanism manipulating the pull-wires to ensure that the catheter always points to the target.

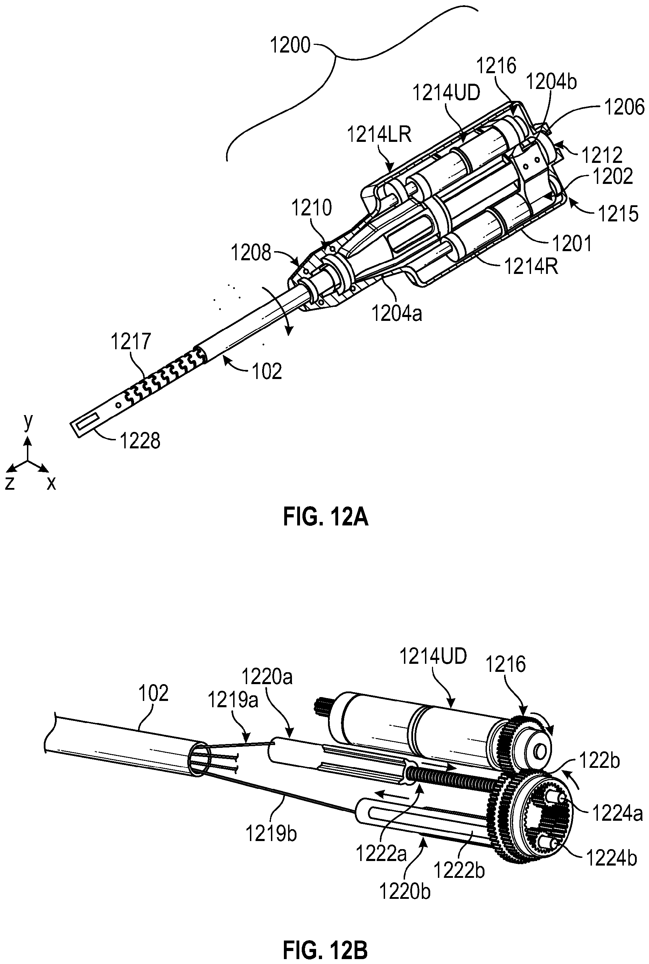

In accordance with the disclosure, the drive mechanism receives signals derived by the computing device 122 to drive the catheter (e.g., extend and retract pull-wires) based on the observed movement of the catheter 102 and the target caused by respiration and cardiac cycles. One example of such a device can be seen in FIG. 12A which depicts a housing including three drive motors to manipulate a catheter extending therefrom in 5 degrees of freedom (e.g., left right, up, down, and rotation). Other types of drive mechanisms including fewer or more degrees of freedom and other manipulation techniques may be employed without departing from the scope of the disclosure.

As noted above, FIG. 12 depicts a drive mechanism 1200 housed in a body 1201 and mounted on a bracket 1202 which integrally connects to the body 1201. The catheter 102 connects to and in one embodiment forms an integrated unit with internal casings 1204 a and 1204b, and connects to a spur gear 1206. This integrated unit is, in one embodiment rotatable in relation to the housing 1201, such that the catheter 102, internal casings 1204 a-b, and spur gear 1206 can rotate about shaft axis "z". The catheter 102 and integrated internal casings 1204 a-b are supported radially by bearings 1208, 1210, and 1212. Though drive mechanism 1200 is described in detail here, other drive mechanisms may be employed to enable a robot or a clinician to drive the catheter to a desired location without departing from the scope of the disclosure.

An electric motor 1214R, may include an encoder for converting mechanical motion into electrical signals and providing feedback to the computing device 122. Further, the electric motor 1214R (R indicates this motor if for inducing rotation of the catheter 102) may include an optional gear box for increasing or reducing the rotational speed of an attached spur gear 1215 mounted on a shaft driven by the electric motor 1214R. Electric motors 1214LR (LR referring to left-right movement of an articulating portion 1217 of the catheter 102) and 1214UD (referring to up-down movement of the articulating portion 1217), each motor optionally includes an encoder and a gearbox. Respective spur gears 1216 and 1218 drive up-down and left-right steering cables, as will be described in greater detail below. All three electric motors 1214 R, LR, and UD are securely attached to the stationary frame 1202, to prevent their rotation and enable the spur gears 1215, 1216, and 1218 to be driven by the electric motors.

FIG. 12B depicts details of the mechanism causing articulating portion 1217 of catheter 102 to articulate. Specifically, the following depicts the manner in which the up-down articulation is contemplated in one aspect of the disclosure. Such a system alone, coupled with the electric motor 1214UD for driving the spur gear 1216 would accomplish articulation as described above in a two-wire system. However, where a four-wire system is contemplated, a second system identical to that described immediately hereafter, can be employed to drive the left-right cables. Accordingly, for ease of understanding just one of the systems is described herein, with the understanding that one of skill in the art would readily understand how to employ a second such system in a four-wire system. Those of skill in the art will recognize that other mechanisms can be employed to enable the articulation of a distal portion of a catheter and other articulating catheters may be employed without departing from the scope of the disclosure.

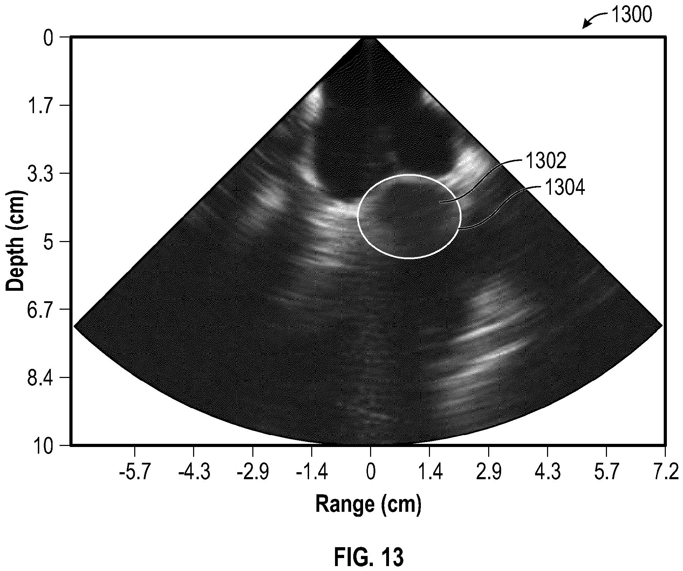

To accomplish up-down articulation of the articulating portion 1217 of the catheter 102, steering cables 1219 a-b may be employed. The distal ends of the steering cables 1219 a-b are attached to, or at, or near the distal end of the catheter 102. The proximal ends of the steering cables 1219 a-b are attached to the distal tips of the posts 1220 a, and 1220 b. As shown in FIG. 13, the posts 1220 a and 1220 b reciprocate longitudinally, and in opposing directions. Movement of the posts 1220 a causes one steering cable 1219 a to lengthen and at the same time, opposing longitudinal movement of post 1220 b causes cable 1219 b to effectively shorten. The combined effect of the change in effective length of the steering cables 1219 a-b is to cause joints a forming the articulating portion 1217 of catheter 102 shaft to be compressed on the side in which the cable 1219 b is shortened, and to elongate on the side in which steering cable 1219 a is lengthened.

The opposing posts 1220 a and 1220 b have internal left-handed and right-handed threads, respectively, at least at their proximal ends. As shown in FIG. 13 housed within casing 1204 b are two threaded shafts 1222 a and 1222 b, one is left-hand threaded and one right-hand threaded, to correspond and mate with posts 1220 a and 1220 b. The shafts 1222 a and 1222 b have distal ends which thread into the interior of posts 1220a and 1220a and proximal ends with spur gears 1224a and 1224bb. The shafts 1222 a and 1222 b have freedom to rotate about their axes. The spur gears 1224 a and 1224 b engage the internal teeth of planetary gear 1226. The planetary gear 1226 also an external teeth which engage the teeth of spur gear 1218 on the proximal end of electric motor 1214UD.

To articulate the catheter in the upwards direction, a clinician may activate via an activation switch (not shown) for the electric motor 1214UD causing it to rotate the spur gear 1218, which in turn drives the planetary gear 1226. The planetary gear 1226 is connected through the internal gears 1224 a and 1224 b to the shafts 1222 a and 1222 b. The planetary gear 1226 will cause the gears 1224 a and 1224 b to rotate in the same direction. The shafts 1222 a and 1222 b are threaded, and their rotation is transferred by mating threads formed on the inside of posts 1220 a and 1220 b into linear motion of the posts 1220 a and 1220 b. However, because the internal threads of post 1220 a are opposite that of post 1220 b, one post will travel distally and one will travel proximally (i.e., in opposite directions) upon rotation of the planetary gear 1226. Thus, the upper cable 1219 a is pulled proximally to lift the catheter 102, while the lower cable 1219 b must be relaxed. As stated above, this same system can be used to control left-right movement of the end effector, using the electric motor 1214LR, its spur gear 1216, a second planetary gear (not shown), and a second set of threaded shafts 1222 and posts 1220 and two more steering cables 1219. Moreover, by acting in unison, a system employing four steering cables can approximate the movements of the human wrist by having the three electric motors 1214 and their associated gearing and steering cables 1219 computer controlled by the computing device 122.

Though generally described above with respect to receiving manual inputs from a clinician as might be the case where the drive mechanism is part of a hand-held catheter system, the disclosure is not so limited. In a further embodiment, the drive mechanism 1200 is part of a robotic system for navigating the catheter 102 to a desired location within the body. In accordance with this disclosure, in instances where the drive mechanism is part of a robotic catheter drive system, the position of the distal portion of the catheter 102 may be robotically controlled. In such an instance the computing device 122, which determines the position of the target and the catheter 102

The drive mechanism may receive inputs from computing device 122 or another mechanism through which the surgeon specifies the desired action of the catheter 102. Where the clinician controls the movement of the catheter 102, this control may be enabled by a directional button, a joystick such as a thumb operated joystick, a toggle, a pressure sensor, a switch, a trackball, a dial, an optical sensor, and any combination thereof. The computing device responds to the user commands by sending control signals to the motors 1214. The encoders of the motors 1214 provide feedback to the control unit 24 about the current status of the motors 1214.

In a further aspect of the disclosure the catheter 102 may include or be configured to receive an ultrasound imager 1228. The ultrasound imager 1228 may be a radial ultrasound transducer, a linear ultrasound transducer, a capacitive micromachined ultrasonic transducer, a piezoelectric micromachined ultrasonic transducers, or others without departing from the scope of the disclosure. In accordance with the disclosure, following the navigation of the catheter 102 to a location proximate the target and conducting the local registration (i.e., the steps of Figs. 3-9), an ultrasound imaging application may be engaged. By conducting the local registration procedure CT-to-body divergence has been eliminated, and the clinician has confidence that the relative position of the catheter 102 and the target as displayed in the navigation software (e.g., Fig. 9) is an accurate representation of the placement of the catheter 102 within the body, relative to the target.

The ultrasound imaging application, when engaged, begins to capture ultrasound images such as image 1300, as depicted in Fig. 13. If local registration has been conducted, the catheter 102, and the ultrasound transducer 1228 will be pointed in the direction of the target 1302. Even without local registration, so long as the catheter 102 is proximate the target as shown in Fig. 2, the target 1302 should be in the field of view in the ultrasound image 1300 taken by the imager 1228. The ultrasound imaging application may request the user to identify target 1302, by for example placing a ring 1304 around the target. Alternatively, the ultrasound imaging application may perform an image an image analysis to identify the target in the image. In either event, once the target 1302 is identified in the field of view, and with the position of the catheter 102 being provided by the sensor 104 or 126, ultrasound imaging application can determine where in the field of view the target 1302 is located and in combination with the computing device 122 generate signals to drive the catheter 102 such that the target 1302 is substantially centered in the field of view. This image analysis of the location of the target 1304 in the field of view and the driving of the catheter 102 to retain the target in the ultrasound image 1300 field of view provides confidence to the clinician that end of the catheter 102 remains aligned with the target despite the effects of respiration, cardiac function, or the passage of tools through the catheter 102. Though shown in Fig. 12B as formed on the catheter 102, the ultrasound transducer 1228 may be formed on a separate catheter, for example the catheter on which the LG sensor 104 is located.

Reference is now made to FIG. 1, which is a schematic diagram of a system 1400 configured for use with the methods of the disclosure including the method of FIG. 11. System 1400 may include a workstation 1401, and optionally a fluoroscopic imaging device or fluoroscope 1415. In some embodiments, workstation 1401 may be coupled with fluoroscope 1415, directly or indirectly, e.g., by wireless communication. Workstation 1401 may include a memory 1402, a processor 1404, a display 1406 and an input device 1410. Processor or hardware processor 1404 may include one or more hardware processors. Workstation 1401 may optionally include an output module 1412 and a network interface 1408. Memory 1402 may store an application 1418 and image data 1414. Application 1418 may include instructions executable by processor 1404 for executing the methods of the disclosure including the method of FIG. 11.

Application 1418 may further include a user interface 1416. Image data 1414 may include the CT scans, the generated fluoroscopic 3D reconstructions of the target area and/or any other fluoroscopic image data and/or the generated one or more slices of the 3D reconstruction. Processor 1404 may be coupled with memory 1402, display 1406, input device 1410, output module 1412, network interface 1408 and fluoroscope 1415. Workstation 1401 may be a stationary computing device, such as a personal computer, or a portable computing device such as a tablet computer. Workstation 1401 may embed a plurality of computer devices.

Memory 1402 may include any non-transitory computer-readable storage media for storing data and/or software including instructions that are executable by processor 1404 and which control the operation of workstation 1401 and, in some embodiments, may also control the operation of fluoroscope 1415. Fluoroscope 1415 may be used to capture a sequence of fluoroscopic images based on which the fluoroscopic 3D reconstruction is generated and to capture a live 2D fluoroscopic view according to this disclosure. In an embodiment, memory 1402 may include one or more storage devices such as solid-state storage devices, e.g., flash memory chips. Alternatively, or in addition to the one or more solid-state storage devices, memory 1402 may include one or more mass storage devices connected to the processor 1404 through a mass storage controller (not shown) and a communications bus (not shown).

Although the description of computer-readable media contained herein refers to solid-state storage, it should be appreciated by those skilled in the art that computer-readable storage media can be any available media that can be accessed by the processor 1404. That is, computer readable storage media may include non-transitory, volatile and non-volatile, removable and non-removable media implemented in any method or technology for storage of information such as computer-readable instructions, data structures, program modules or other data. For example, computer-readable storage media may include RAM, ROM, EPROM, EEPROM, flash memory or other solid-state memory technology, CD-ROM, DVD, Blu-Ray or other optical storage, magnetic cassettes, magnetic tape, magnetic disk storage or other magnetic storage devices, or any other medium which may be used to store the desired information, and which may be accessed by workstation 1001.

Application 1418 may, when executed by processor 1404, cause display 1406 to present user interface 1416. User interface 1416 may be configured to present to the user a single screen including a three-dimensional (3D) view of a 3D model of a target from the perspective of a tip of a medical device, a live two-dimensional (2D) fluoroscopic view showing the medical device, and a target mark, which corresponds to the 3D model of the target, overlaid on the live 2D fluoroscopic view, as shown, for example, in FIG. 2. User interface 1416 may be further configured to display the target mark in different colors depending on whether the medical device tip is aligned with the target in three dimensions.

Network interface 1408 may be configured to connect to a network such as a local area network (LAN) consisting of a wired network and/or a wireless network, a wide area network (WAN), a wireless mobile network, a Bluetooth network, and/or the Internet. Network interface 1408 may be used to connect between workstation 1401 and fluoroscope 1415. Network interface 1408 may be also used to receive image data 1414. Input device 1410 may be any device by which a user may interact with workstation 1401, such as, for example, a mouse, keyboard, foot pedal, touch screen, and/or voice interface. Output module 1412 may include any connectivity port or bus, such as, for example, parallel ports, serial ports, universal serial busses (USB), or any other similar connectivity port known to those skilled in the art. From the foregoing and with reference to the various figures, those skilled in the art will appreciate that certain modifications can be made to the disclosure without departing from the scope of the disclosure.

While detailed embodiments are disclosed herein, the disclosed embodiments are merely examples of the disclosure, which may be embodied in various forms and aspects. For example, embodiments of an electromagnetic navigation system, which incorporates the target overlay systems and methods, are disclosed herein; however, the target overlay systems and methods may be applied to other navigation or tracking systems or methods known to those skilled in the art. Therefore, specific structural and functional details disclosed herein are not to be interpreted as limiting, but merely as a basis for the claims and as a representative basis for teaching one skilled in the art to variously employ the disclosure in virtually any appropriately detailed structure.

The invention may be described by reference to the following numbered paragraphs:-
1. A method of maintaining a position of a catheter comprising:
   determining a position of a distal end of the catheter;
   determining a position of a target;
   calculating an offset between the distal end of the catheter and the target;
   monitoring movement of the target and catheter;
   sending signals to one or more motors, the motors being in operable communication with and configured to adjust a distal portion of the catheter; and
   driving the one or more motors such that the offset between the distal end of the catheter and the target is substantially maintained.
2. The method of paragraph 1, further comprising conducting a local registration of the catheter and the target.
3. The method of paragraph 2, further comprising capturing a fluoroscopic image of the target and the catheter.
4. The method of paragraph 3, further comprising comparing the fluoroscopic image to slices of a fluoroscopic 3D reconstruction captured during the local registration.
5. The method of paragraph 1, further comprising detecting the position of the target using ultrasonic visualization.
6. The method of paragraph 1, further comprising detecting a position of a distal end of the catheter with a sensor.
7. The method of paragraph 5, wherein the position of the target is calculated based on the calculated offset and the detected position of the distal end of the catheter.
8. A method of navigating a catheter to a desired location within a luminal network comprising:
   receiving a CT image data set;
   identifying one or more targets in the CT image data set;
   generating a three-dimensional (3D) model of the luminal network and pathway to the one or more targets;
   registering the 3D model and pathway to a luminal network;
   updating the position of the catheter in the 3D model as the along the pathway proximate one of the targets;
   performing a local registration to determine a relative position of the catheter and one of the targets;
   acquiring a fluoroscopic image of the luminal network;
   determining a position of the target and the catheter in the fluoroscopic image;
   detecting a position of a sensor on a distal portion of the catheter;
   calculating a position of the target in a coordinate system of the sensor; and
   receiving signals from a computing device to drive one or more motors operably associated with the catheter to maintain the catheter position relative to the target position constant.
9. The method of paragraph 8, further comprising detecting the position of the target using ultrasonic visualization.
10. The method of paragraph 8, wherein the sensor is operably connected to a distal portion of a catheter.
11. The method of paragraph 10, wherein the sensor is an electromagnetic sensor.
12. The method of paragraph 10, wherein the position of the target is calculated based on the calculated offset and the detected position of the distal end of the catheter.
13. The method of paragraph 11, further comprising detecting a position of one or more reference sensors.
14. The method of paragraph 13, wherein the fluoroscopic image is a fluoroscopic video, and the calculating a position of the target in a coordinate system of the sensor in undertaken multiple times through the fluoroscopic video.
15. The method of paragraph 14, further comprising matching movement of the target determined by repeatedly determining of the target's position with the movement of the one or more reference sensor and the sensor on the distal portion of the catheter.
16. The method of paragraph 15, further comprising driving the one or more motors based on the received driving signals to maintain the catheter position relative to the target position constant when the movement of the one or more reference sensor or the sensor on the distal portion of the catheter is within a tolerance limit.
17. The method of paragraph 15, further comprising detecting that the position of the reference sensor or sensor on the distal portion of the catheter is outside of a tolerance limit;
   performing a second local registration to determine a new relative position of the catheter and one of the targets;
   acquiring a further fluoroscopic image of the luminal network;
   determining a new position of the target and the catheter in the fluoroscopic image;
   detecting a new position of a sensor on a distal portion of the catheter;
   calculating a new position of the target in a coordinate system of the sensor; and
   receiving signals from a computing device to drive one or more motors operably associated with the catheter to maintain the new catheter position relative to the new target position constant.
18. A system comprising:
   a catheter including a drive mechanism to manipulate a distal portion of the catheter, the catheter including a sensor on a distal portion thereof;
   a computing device including a processor and a computer readable recording medium, the computing device configured to:
      receive signals from the sensor to determine a position of the distal portion of the catheter;
      receive signals from one or more reference sensors;
      receive a fluoroscopic video of the catheter proximate a target;
      determine a relative position of the catheter and the target at multiple instances in the fluoroscopic video;
      calculate a position of the target in a coordinate system of the sensor; and
      generate signals to drive the distal portion of the catheter to maintain the catheter position relative to the target position constant.
19. The system of paragraph 18, wherein the drive mechanism includes one or more motors operably connected to one or more pull wires and configured to receive the signals generated by the computing device.
20. The system of paragraph 18, where in the sensor on the distal portion of the catheter is an electromagnetic sensor.

## Claims

1. A method of maintaining a position of a catheter comprising:
determining a position of a distal end of the catheter;
determining a position of a target;
calculating an offset between the distal end of the catheter and the target;
monitoring movement of the target and catheter;
sending signals to one or more motors, the motors being in operable communication with and configured to adjust a distal portion of the catheter; and
driving the one or more motors such that the offset between the distal end of the catheter and the target is substantially maintained.

2. The method of claim 1, further comprising conducting a local registration of the catheter and the target; preferably further comprising capturing a fluoroscopic image of the target and the catheter.

3. The method of claim 2, further comprising comparing the fluoroscopic image to slices of a fluoroscopic 3D reconstruction captured during the local registration.

4. The method of any preceding claim, further comprising detecting the position of the target using ultrasonic visualization.

5. The method of any preceding claim, further comprising detecting a position of a distal end of the catheter with a sensor; preferably wherein the position of the target is calculated based on the calculated offset and the detected position of the distal end of the catheter.

6. A method of navigating a catheter to a desired location within a luminal network comprising:
receiving a CT image data set;
identifying one or more targets in the CT image data set;
generating a three-dimensional (3D) model of the luminal network and pathway to the one or more targets;
registering the 3D model and pathway to a luminal network;
updating the position of the catheter in the 3D model as the along the pathway proximate one of the targets;
performing a local registration to determine a relative position of the catheter and one of the targets;
acquiring a fluoroscopic image of the luminal network;
determining a position of the target and the catheter in the fluoroscopic image;
detecting a position of a sensor on a distal portion of the catheter;
calculating a position of the target in a coordinate system of the sensor; and
receiving signals from a computing device to drive one or more motors operably associated with the catheter to maintain the catheter position relative to the target position constant.

7. The method of claim 6, further comprising detecting the position of the target using ultrasonic visualization.

8. The method of claim 6 or claim 7, wherein the sensor is operably connected to a distal portion of a catheter.

9. The method of claim 8, wherein the sensor is an electromagnetic sensor; preferably wherein the position of the target is calculated based on the calculated offset and the detected position of the distal end of the catheter.

10. The method of claim 9, further comprising detecting a position of one or more reference sensors.

11. The method of claim 10, wherein the fluoroscopic image is a fluoroscopic video, and the calculating a position of the target in a coordinate system of the sensor in undertaken multiple times through the fluoroscopic video; preferably further comprising matching movement of the target determined by repeatedly determining of the target's position with the movement of the one or more reference sensor and the sensor on the distal portion of the catheter.

12. The method of claim 11, further comprising driving the one or more motors based on the received driving signals to maintain the catheter position relative to the target position constant when the movement of the one or more reference sensor or the sensor on the distal portion of the catheter is within a tolerance limit.

13. The method of claim 12, further comprising detecting that the position of the reference sensor or sensor on the distal portion of the catheter is outside of a tolerance limit;
performing a second local registration to determine a new relative position of the catheter and one of the targets;
acquiring a further fluoroscopic image of the luminal network;
determining a new position of the target and the catheter in the fluoroscopic image;
detecting a new position of a sensor on a distal portion of the catheter;
calculating a new position of the target in a coordinate system of the sensor; and
receiving signals from a computing device to drive one or more motors operably associated with the catheter to maintain the new catheter position relative to the new target position constant.

14. A system comprising:
a catheter including a drive mechanism to manipulate a distal portion of the catheter, the catheter including a sensor on a distal portion thereof;
a computing device including a processor and a computer readable recording medium, the computing device configured to:
receive signals from the sensor to determine a position of the distal portion of the catheter;
receive signals from one or more reference sensors;
receive a fluoroscopic video of the catheter proximate a target;
determine a relative position of the catheter and the target at multiple instances in the fluoroscopic video;
calculate a position of the target in a coordinate system of the sensor; and
generate signals to drive the distal portion of the catheter to maintain the catheter position relative to the target position constant.

15. The system of claim 14, wherein the drive mechanism includes one or more motors operably connected to one or more pull wires and configured to receive the signals generated by the computing device preferably wherein the sensor on the distal portion of the catheter is an electromagnetic sensor.
